Europäisches Patentamt

European Patent Office

Office européen des brevets

(19)

(11) Veröffentlichungsnummer: **0 356 683**

**A1**

# EUROPÄISCHE PATENTANMELDUNG

(21) Anmeldenummer: **89113139.3**

(22) Anmeldetag: **18.07.89**

(51) Int. Cl.5: **A61M 25/02**

(30) Priorität: **04.08.88 DE 8809933 U**

(43) Veröffentlichungstag der Anmeldung:
**07.03.90 Patentblatt 90/10**

(84) Benannte Vertragsstaaten:
**AT BE CH DE ES FR GB GR IT LI LU NL SE**

(71) Anmelder: **Fresenius AG**
**Gluckensteinweg 5**
**D-6380 Bad Homburg v.d.H.(DE)**

(72) Erfinder: **Krütten, Viktor**
**Richard Klingel Strasse 27**
**D-6270 Idstein(DE)**

(74) Vertreter: **Görtz, Dr. Fuchs, Dr. Luderschmidt**
**Patentanwälte**
**Abraham-Lincoln-Strasse 7 Postfach 46 60**
**D-6200 Wiesbaden(DE)**

(54) **Befestigungseinrichtung, insbesondere für perkutan applizierte Leitungen, wie Katheter, Drains, Schläuche und ähnlichem auf der Haut des Patienten.**

(57) Die Neuerung betrifft eine Befestigungseinrichtung, insbesondere für perkutanapplizierte Leitungen, wie Katheter, Drains, Schläuche und ähnlichem auf der Haut des Patienten, ohne daß das Lumen der Leitung (9) verändert wird. Die Befestigungseinrichtung weist eine Befestigungsplatte (1) und ein darin einsetzbares separates Klemmteil (4) auf, das mit Hintergreifungselementen (6) versehen ist. Das Klemmteil (4) ist beim Einrasten der Hintergreifungselemente (6) mit der Befestigungsplatte (1) verbunden und umschließt ein Schlauchteil (9) bzw. ein Quetschteil (7).

FIG.1

EP 0 356 683 A1

## Befestigungseinrichtung, insbesondere für perkutanapplizierte Leitungen, wie Katheter, Drains, Schläuche und ähnlichem auf der Haut des Patienten

Die Neuerung betrifft eine Befestigungseinrichtung, insbesondere für perkutanapplizierte Leitungen, wie Katheter, Drains, Schläuche und ähnlichem auf der Haut des Patienten, ohne daß das Lumen verändert wird. Solche Leitungen werden insbesondere für suprapubische Harnableitungen sowie als Nephrostomie-Katheter verwendet. Derartige Befestigungen werden im medizinischen Bereich insbesondere zur Fixation von Schläuchen, Drains und Kathetern eingesetzt. Besonders bei suprapubischen Harnableitungen sowie Nephrostomie-Kathetern besteht die Schwierigkeit, den aus dem Körper austretenden Katheter, Drain, Schlauch usw. so zu befestigen, daß keine Behinderungen des Patienten eintreten. Bisher wurden die Leitungen bzw. Schläuche auf die Haut des Patienten genäht, wobei insbesondere durch die Körperbewegungen des Patienten erhebliche Behinderungen auftraten und eine individuelle Anpassung an verschiedene Eindringtiefen der Schläuche verhinderte. Dabei kam es immer wieder zu Abrissen der Leitungen von der Haut des Patienten, da sich die Leitungen gegenüber der Annähstelle nicht lageverändern konnten. Andere Leitungen wiederum wurden direkt im Körper lagefixiert, was ebenfalls erhebliche Schwierigkeiten mit sich brachte.

Die Aufgabe der Neuerung bestand deshalb darin, eine Möglichkeit einer Befestigungseinrichtung zu schaffen, die nicht invasiv und doch eine sichere Fixation des austretenden Katheters, Drains, Schlauches oder ähnlichem ermöglicht und ein Abknicken verhindert.

Neuerungsgemäß wird die Aufgabe gelöst durch eine Befestigungsplatte und ein darin einsetzbares separates Klemmteil, das mit Hintergreifungselementen versehen ist, wobei das Klemmteil beim Einrasten der Hintergreifungselemente mit der Befestigungsplatte verbunden ist und ein Schlauchteil umschließt.

Durch diese neuerungsgemäße Lösung wird erreicht, daß die Befestigungseinrichtung alternativ durch nicht-invasiven Adhesiverband oder bisher gebräuchlicher Nahtbefestigung sicher appliziert werden kann.

Dazu weist die Befestigungsplatte Durchtrittsöffnungen zum Annähen der Befestigungsplatte auf der Haut des Patienten auf. Weiterhin ist ein Quetschteil vorhanden, das sowohl separat als auch an der Befestigungsplatte integriert ausgebildet sein kann. Das Quetschteil ist über die Leitung gezogen und zumindest teilweise von dem Klemmteil umschlossen. Dabei ist das Quetschtteil mittels eines axialen Längsschnittes im Durchmesser variierbar ausgebildet.

Das Klemmteil weist Hintergreifungselemente auf, die sich gegenseitig hintergreifen und selbständig den Klemmechanismus bilden.

In einer anderen Ausführungsform weist das Klemmteil Hintergreifungselemente auf, die in Hintergreifungselemente an der Befestigungsplatte eingreifen.

Im Ergebnis der Neuerung ist es möglich, zwar die Befestigungseinrichtung auf der Haut des Patienten zu fixieren, aber durch Bedienen des Klemmteils eine Schlauchverschiebung zu gewährleisten und eine axiale Verschiebbarkeit der Leitung zur Befestigungseinrichtung zu ermöglichen.

An zwei Ausführungsbeispielen soll die Neuerung näher erläutert werden. Die zugehörigen Zeichnungen zeigen in

Figur 1 eine erste neuerungsgemäße Ausbildungsform einer Befestigungseinrichtung in der Vorderansicht;

Figur 2 einen Schnitt A-A gemäß der Ausbildungsform nach Figur 1;

Figur 3 die Vorderansicht einer weiteren Ausbildungsform und

Figur 4 einen Schnitt entlang der Linie B-B gemäß Figur 3.

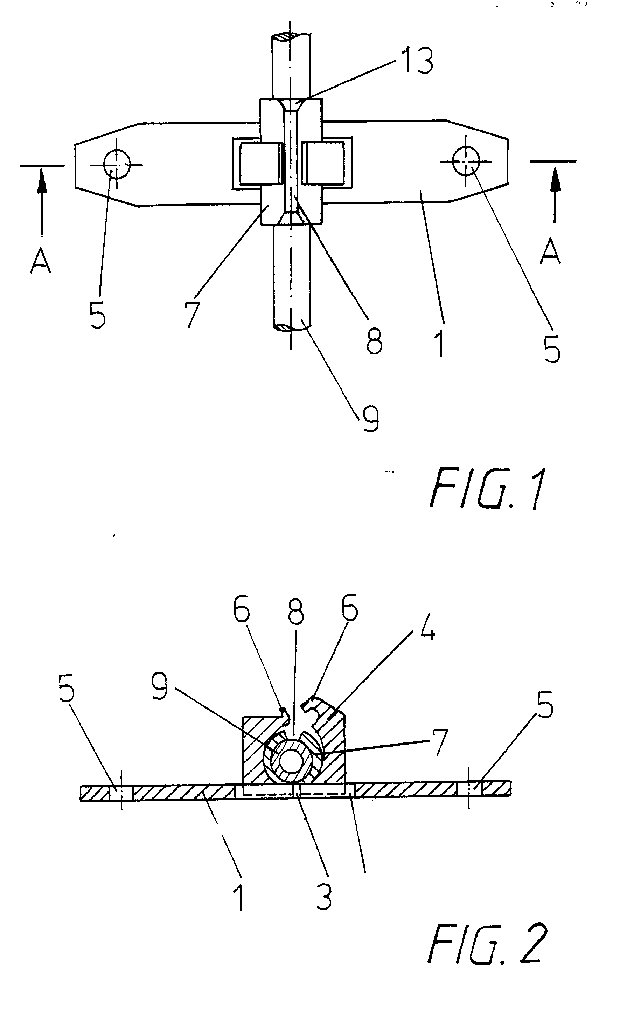

Die Figuren 1 und 2 zeigen eine Befestigungseinrichtung, bei der das Klemmteil Hintergreifungselemente aufweist, die sich gegenseitig hintergreifen und selbständig den Klemmechanismus bilden. Die Befestigungseinrichtung weist eine Befestigungsplatte 1 auf, die einen Durchbruch 2 mit Mittelstegen 3 zur Aufnahme des Klemmteils 4 besitzt. Im Bereich der äußeren Enden der Befestigungsplatte 1 sind Durchtrittsbohrungen 5 vorgesehen, mittels denen die Befestigungsplatte 1 auf der Haut des Patienten festgenäht werden kann. Die Befestigungsplatte 1 besteht wie alle anderen Einzelteile der Befestigungseinrichtung aus einem relativ weichen Kunststoff.

Das Klemmteil 4 wird im geöffneten Zustand von hinten durch den Durchbruch 2 der Befestigungsplatte 1 hindurchgeführt und durch die Mittelstege 3, die in entsprechende Ausnehmungen (nicht dargestellt) des Klemmteils 4 eingreifen. An den oberen offenen, sich gegenüberliegenden Enden des Klemmteils 4 sind Hintergreifungselemente 6 vorgesehen, die zum Verschließen des Klemmteils 4 benutzt werden und einen Nut- und Federmechanismus bilden, der nach Verschließen nur durch gezielte Manipulation wieder geöffnet werden kann. Diese Hintergreifungselemente 6 sind haken- bzw. nasenförmig ausgebildet.

Weiterhin ist ein Quetschteil 7 vorgesehen, das sowohl als separates Einzelteil, welches in die Höhlung des Klemmteils 4 eingeschoben wird, oder als integrierter Bestandteil der Befestigungsplatte 1 ausgebildet sein kann. In letzterem Fall ist das Quetschteil 7 fest mit der Befestigungsplatte 1 verbunden.

In einer vorteilhaften Ausbildungsform kann das Quetschteil 7 in seiner axialen Länge auf einer Seite geschnitten sein (8). Dadurch kann dessen Durchmesser für die Aufnahme von Schläuchen unterschiedlicher Durchmesser variiert werden. Nachdem die entsprechende Leitung des Katheters, Drains oder Schlauches in das Quetschteil 7 eingelegt worden ist, wird das Klemmteil 4 über die Hintergreifungselemente 6 durch einfaches Zusammendrücken verschlossen. Dabei wird das Quetschteil 7 durch das Klemmteil 4 zusammengedrückt und legt sich wiederum um den Schlauch 9. Dadurch wird der Schlauch an der Befestigungseinrichtung fixiert. Am Klemmteil 4 können nicht näher dargestellte Griffnuten eingearbeitet sein, die ein besseres Greifen zum Verschließen des Klemmteils 4 ermöglichen. Die Befestigungseinrichtung besitzt im nicht geschlossenen Zustand des Klemmteils 4 ein so großes Spiel, das ein axiales Verschieben der Befestigungseinrichtung auf der Leitung möglich ist.

Das Quetschteil 7 ist zirkulär umlaufend mit einer Fase 13 versehen, die ein Abknicken an der Austrittsstelle des Schlauches 9 verhindert.

Es sei noch darauf hingewiesen, daß die Ausführungsform gemäß Figuren 1 und 2 im geöffneten Zustand des Klemmteils 4 dargestellt ist.

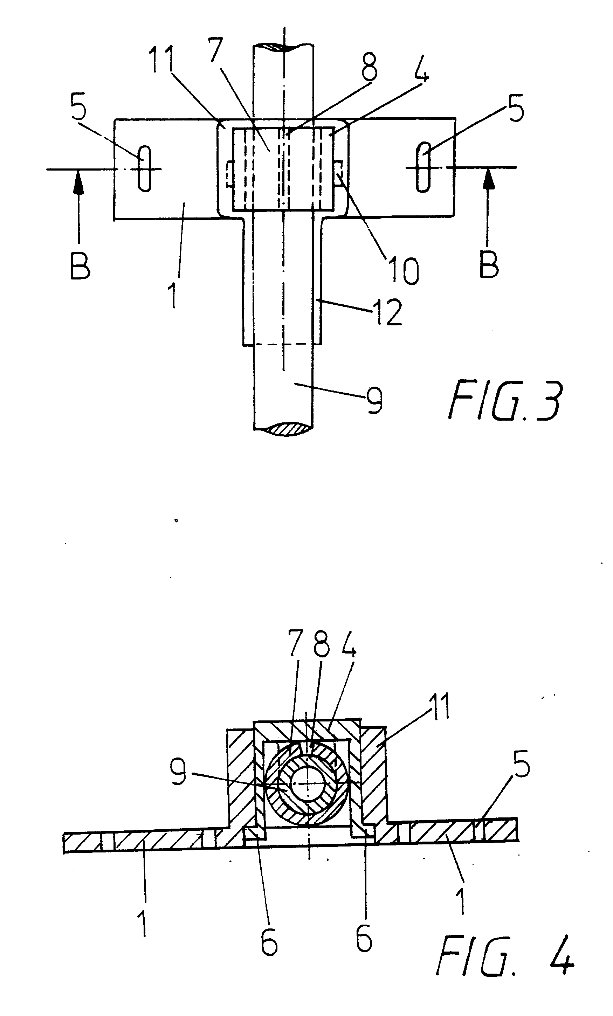

Die Figuren 3 und 4 zeigen eine weitere Ausbildungsform, bei der das Klemmteil 4 in geschlossenem Zustand dargestellt ist und Hintergreifungselemente 6 aufweist, die in Hintergreifungselemente 10 an der Befestigungsplatte 1 eingreifen. Diese Hintergreifungselemente 10 sind in einer kastenartigen, mit der Befestigungsplatte 1 integrierten Erhöhung 11 angeordnet. In diese kastenartige Erhöhung 11 ist ein vorzugsweise mit einem axialen Längsschnitt 8 versehenes Quetschteil 7 eingelegt, das den zu klemmenden Schlauch 9 aufnimmt. An wenigstens einer Seite der kastenartigen Erhöhung 11, bezogen auf die Schlauchführung, kann eine verlängerte Führungsnut 12 integriert sein, in der der Schlauch 9 liegt und die ein Abknicken des Schlauches 9 verhindern soll. Das Klemmteil 4 ist U-förmig ausgebildet und weist an den sich gegenüberliegenden Schenkeln Hintergreifungselemente 6 auf. Das U-förmige Klemmteil wird nun mit seiner Aushöhlung über das Quetschteil 4 geschoben, wobei die Hintergreifungselemente 6 des Klemmteils 4 in die Hintergreifungselemente 10 der kastenförmigen Erhöhung 11 eingreifen. Dadurch wird das Quetschteil 7 zusammengedrückt und übt seinerseits eine Kompression auf den Schlauch 9 auf. Damit ist der Schlauch 9 lagefixiert. Durch gezielte Manipulation kann das Klemmelement 4 wieder von der Befestigungsplatte 1 entfernt werden.

Durch das einfache Betätigen des Klemmteils zur Fixation des Schlauches bzw. zum Freigeben des Schlauches aus dem Befestigungselement kann die Lageänderung des Schlauches am Körper des Patienten ständig bei Bedarf variiert werden. Durch die flache Ausbildung der Befestigungsplatte und deren Bestehen aus einem weichem Kunststoff wird ermöglicht, daß sich die Befestigungsplatte optimal an Körperrundungen anpaßt. Der Schlauch wird nicht mehr invasiv angebracht und ermöglicht dennoch eine sichere Fixation des austretenden Katheters oder Drains und verhindert ein Abknicken der Leitung.

**Ansprüche**

1. Befestigungseinrichtung, insbesondere für perkutanapplizierte Leitungen, wie Katheter, Drains, Schläuche u.ä. auf der Haut eines Patienten, gekennzeichnet durch eine auf der Haut des Patienten zu befestigende Befestigungsplatte (1), ein auf der Befestigungsplatte angeordnetes, hülsenartiges, axial geschlitztes Quetschteil (7) für die Aufnahme bzw. das Durchführen eines Schlauches, und ein das Quetschteil zumindest teilweise umgebendes Klemmteil (4), welches mit Hintergreifungselementen (6) versehen und unter Ausübung einer Druckkraft auf das Quetschteil (7) mittels seiner Hintergreifungselemente (6) verrastbar ist.

2. Befestigungseinrichtung nach Anspruch 1, dadurch gekennzeichnet, daß das Quetschteil (7) mit der Befestigungsplatte (1) in vorzugsweise einstückiger Ausführung verbunden ist.

3. Befestigungseinrichtung nach Anspruch 2, dadurch gekennzeichnet, daß das Quetschteil (7) im wesentlichen entlang einer axialen äußeren Mantellinie mit der Befestigungsplatte (1) verbunden ist.

4. Befestigungseinrichtung nach mindestens einem der Ansprüche 1 bis 3, dadurch gekennzeichnet, daß das Klemmteil (4) in der Befestigungsplatte (1) gelagert ist und zwei das Quetschteil (7) von unten umgreifende Backen aufweist, die an ihren der Befestigungsplatte abgewandten Enden mit den Hintergreifungselementen (6) versehen und unter Preßung des Quetschteiles (7) um dieses herum mittels der Hintergreifungselemente (6) gegenseitig verriegelbar sind.

5. Befestigungseinrichtung nach Anspruch 4, dadurch gekennzeichnet, daß die Backen des Klemmteiles (4) getrennt in Durchbrüchen (2) der Befestigungsplatte (1) zu beiden Seiten des Quetschteiles (7) gelagert sind.

6. Befestigungseinrichtung nach Anspruch 5, dadurch gekennzeichnet, daß die Backen des KLemmteiles (4) durch ein Joch miteinander verbunden sind und daß die Befestigungsplatte (1) seitlich des Quetschteiles (7) mit Durchbrüchen (2) versehen ist, durch die das Klemmteil (4) mit seinen Backen von der dem Quetschteil (7) abgewandten Seite her durch die Befestigungsplatte (1) hindurchführbar sind.

7. Befestigungseinrichtung nach Anspruch 1, dadurch gekennzeichnet, daß das Klemmteil (4) im wesentlichen U-förmig ausgebildet und an den Enden seiner Schenkel mit den Hintergreifungselementen (6) versehen ist, mit denen es von oben das Quetschteil (7) umgebend mit Gegenelementen in der Befestigungsplatte (1) verrastbar ist

8. Befestigungseinrichtung nach Anspruch 7, dadurch gekennzeichnet, daß das Quetschteil (7) auf der Befestigungsplatte (1) innerhalb einer oben offenen und mit stirnseitigen Durchtrittsöffnungen für einen Schlauch versehenen, kastenartigen Erhöhung (11) frei angeordnet ist.

9. Befestigungseinrichtung nach Anspruch 7 und 8, dadurch gekennzeichnet, daß die Schenkel des Klemmteiles (4) zwischen das Quetschteil (7) und die Seitenwände der kastenartigen Erhöhung (11) einführbar und am Grund dieser Seitenwände verrastbar sind.

10. Befestigungseinrichtung nach mindestens einem der Ansprüche 1 bis 9, dadurch gekennzeichnet, daß die Befestigungsplatte (1) mit Durchtrittsöffnungen (5) für das Annähen auf der Haut des Patienten versehen ist.

11. Befestigungseinrichtung nach mindestens einem der Ansprüche 1 bis 10, dadurch gekennzeichnet, daß sie aus einem weichen, verformbaren Kunststoff-Material besteht.

*FIG.1*

*FIG. 2*

*FIG.3*

*FIG. 4*

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| Y | US-A-3 834 380 (BOYD)<br>* Spalte 3, Zeilen 3-27; Figur 4 *<br>--- | 1-4,7,<br>10,11 | A 61 M 25/02 |
| Y | DE-C- 831 757 (HEUCKEROTH)<br>* Seite 2, Zeile 97 - Seite 3, Zeilen 13,33-35; Figur 5 *<br>--- | 1-3,7,<br>10,11 | |
| Y | US-A-4 480 639 (PETERSON)<br>* Spalte 3, Zeilen 37-39; Spalte 4, Zeilen 10-22; Figuren 7,8 * | 1-4,10,<br>11 | |
| A | <br>--- | 5 | |
| P,X | DE-U-8 809 933 (FRESENIUS)<br>* Insgesamt *<br>----- | 1-11 | |

| | | | RECHERCHIERTE SACHGEBIETE (Int. Cl.5) |
|---|---|---|---|
| | | | A 61 M |

Der vorliegende Recherchenbericht wurde für alle Patentansprüche erstellt

| Recherchenort | Abschlußdatum der Recherche | Prüfer |
|---|---|---|
| DEN HAAG | 02-11-1989 | KOUSOURETAS I. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
........................................................................
& : Mitglied der gleichen Patentfamilie, übereinstimmendes Dokument